# EUROPEAN PATENT SPECIFICATION

(11) **EP 0 819 666 B1**
(45) Date of publication and mention of the grant of the patent: **10.09.2003**
(21) Application number: 97112023.3
(22) Date of filing: 15.07.1997
(51) Int. Cl.: C07C 15/04, C07C 15/08, C07C 6/12

(54) **Toluene disproportionation process**
Verfahren zur Disproportionierung von Toluol
Procédé de disproportionation de toluène

(30) Priority: 15.07.1996 US 679889; 15.07.1996 US 679886
(43) Date of publication of application: 21.01.1998
(73) Proprietor: FINA TECHNOLOGY, INC., Houston, Texas 77267-4412 (US)
(72) Inventor: Kelly, Kevin P., Friendswoord, Texas 77546 (US); Butler, James R., Houston, Texas 77059 (US)
(74) Representative: Leyder, Francis

(56) References cited:
- US-A- 4 956 511

## Description

### BACKGROUND OF THE INVENTION

This invention relates to the disproportionation of alkylaromatic feedstreams and more particularly to disproportionation of toluene containing feedstocks employing dealuminated nickel mordenite catalysts.

### DESCRIPTION OF THE RELATED ART

Toluene disproportionation (TDP) involves a well known transalkylation reaction in which toluene is converted to benzene and xylene in accordance with the following reaction:

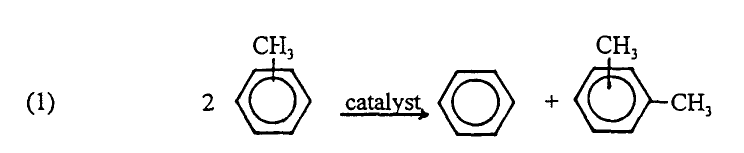

### [Reaction (I) is mildly exothermic.]

Mordenite is one of a number of molecular sieve catalysts useful in the transalkylation of alkylaromatic compounds. Mordenite is a crystalline aluminosilicate zeolite exhibiting a network of silicon and aluminum atoms interlinked by oxygen atoms within the crystalline structure. For a general description of mordenite catalysts, reference is made to Kirk-Othmer, Encyclopedia of Chemical Technology, 3rd Edition, 1981, under the heading "Molecular Sieves", Vol. 15, pages 638-643. Mordenite as found in nature or as synthesized to replicate the naturally occurring zeolite, typically exhibits a relatively low silica-to-alumina mole ratio of about 10 or less. Also known, however, are mordenite catalysts exhibiting a substantially lower alumina content. These alumina deficient mordenite catalysts exhibit silica-to-alumina ratios greater than 10, ranging up to about 100, and may be prepared by direct synthesis as disclosed, for example, in U.S. Patent No. 3,436,174 to *Sand* or by acid extraction of a more conventionally prepared mordenite as disclosed in U.S. Patent No. 3,480,539 to *Voorhies et al*. Both the typical and the aluminum deficient mordenites are known to be useful in the disproportionation of toluene.

Disproportionation of toluene feedstock may be performed at temperatures ranging from about 200°C to about 600°C or above and at pressures ranging from atmospheric to perhaps 100 atmospheres or above and at liquid hourly space velocities (LHSV) of around 2hr⁻². The specific catalyst, however, may impose constraints on reaction temperatures in terms of catalyst activity and aging characteristics. In general, the prior art suggests the use of relatively high temperatures when employing the high aluminum mordenites (low silica-to-alumina ratios) and somewhat lower temperatures when employing the low alumina mordenites. Accordingly, where mordenite catalysts exhibiting high silica/alumina ratios have been employed in the transalkylation of alkylaromatics, it has been the practice to operate toward the lower end of the temperature range. U.S. Patent No. 4,665,258 to *Butler et al*., however, discloses disproportionation of a toluene containing feedstock employing an aluminum deficient mordenite catalyst under relatively severe disproportionation conditions; involving a temperature range of 370° - 500°C. The mordenite catalysts exhibit silica/alumina mole ratios of at least 30 and, more desirably, within the range of 40-60. The feedstock may be supplied to the reaction zone containing the mordenite catalyst at rates providing relatively high space velocities. The toluene weight hourly space velocity (WHSV) may be greater than 1. Hydrogen is supplied to the reaction zone at a hydrogen/toluene mole ratio within the range of 3-6. The hydrogen pressure may be 34.5 bars (500 psi) or more. The toluene feedstock need not be dried before supplying it to the reaction zone and the patent discloses toluene feedstocks exhibiting a water content in excess of 100 ppm.

The *Butler* '258 patent also discloses passing a hot preflush gas, nitrogen or hydrogen, to the reaction zone prior to initiating the disproportionation reaction. The preflush gas is heated to a temperature sufficient to substantially dehydrate the catalyst by the time the toluene feed is started. This measure enables the disproportionation process to initially be performed at a somewhat lower temperature and without reduction in toluene conversion. As the disproportionation proceeds, temperature progressively increases to maintain toluene conversion at the desired level, typically about 80 percent of theoretical.

U.S. Patent No. 4,723,049 to *Menard et al*. discloses toluene disproportionation carried out over aluminum deficient mordenite of the type disclosed in the aforementioned patent to *Butler.* In this process, preferably carried out at a reaction zone temperature of 370° - 500°C, and more preferably at a temperature of 400° - 500°C with an unmodified aluminum deficient mordenite catalyst, the supply of toluene to the reaction zone is interrupted while the supply of hydrogen is continued. Preferably the period of interruption during which hydrogen supply is continued is for at least one day prior to reinstating the supply of toluene feedstock to the reaction zone. This mode of operation is disclosed to enhance the aging quality of the catalyst and show a reduction in reaction zone temperature without a corresponding decrease in toluene conversion.

It is also a common practice to promote an aluminum deficient mordenite catalyst with a catalytically active metallic content. For example, U. S. Patent No. 3,476,821 to *Brandenburg et al*. discloses disproportionation reactions employing mordenite catalysts having silica/alumina ratios within the range of 10-100 and preferably within the range of 20-60. The mordenites are modified by the inclusion of a sulfided metal selected from the Group VIII metals. The metal may be included in the mordenite by well known ion exchange or impregnated techniques. The especially preferred sulfided Group VIII metals are cobalt and nickel present in a concentration of 0.5 - 10 weight percent. When compared with nickel oxide, nickel sulfide is said to provide less overactivity as indicated by gas and saturated hydrocarbon yield. Here the desired temperature ranges are said to be from about 204 - 359° C (400° - 750° F) and preferably 232 - 338° C (450° - 640° F). The metal promoters are said to substantially increase activity and catalyst life, as indicated by runs extending over several hours or days.

As noted previously, hydrogen is supplied along with toluene to the reaction zone. While the disproportionation reaction (1) does not involve chemical consumption of hydrogen, the use of a hydrogen cofeed is generally considered to prolong the useful life of the catalyst, as disclosed, for example, in the above mentioned patent to *Brandenburg*. The amount of hydrogen supplied, which is normally measured in terms of the hydrogen/toluene mole ratio, is generally shown in the prior art to increase as temperature increases.

*Bhavikatti et al*., "Toluene Disproportionation Over Aluminum-Deficient and Metal-Loaded Mordenites. 1. Catalytic Activity and Aging", Ind. Eng. Chem. Prod. Res. Dev. 1981, 20, 102-105, discloses toluene disproportionation at 400°C over mordenite catalysts having silica/alumina mole ratios ranging from 12 to 61 at atmospheric pressure and a space velocity (WHSV) of 1. As the silica/alumina mole ratio is increased, catalyst activity is substantially decreased while aging quality is increased. That is, the aging rates were lower. Based upon short term aging studies, the best silica/alumina mole ratio appeared to be 23. Catalyst decay was also suppressed by loading the mordenites with nickel. Mordenites having a silica/alumina ratio of 12, 16 and 23 were modified by the inclusion of nickel by a procedure involving ion exchanging ammonium mordenite with an aqueous solution of nickel nitrate. After ion exchange, the catalyst was activated under a hydrogen environment for two hours. The best activation temperature for nickel modified mordenite having a silica/alumina ratio of 23 was indicated to be about 550°C. The nickel modified mordenite having a silica/alumina ratio of 12 showed significantly lower activity when compared to the nickel loaded mordenite of a silica/alumina ratio of 23.

Other patents directed to toluene disproportionation catalysts and processes include *Mitsche* U.S. 3,562,345; *Mitsche* U.S. 3,677,973; *Marcilly U.S. 4,151,120; Dufresne et al*. U.S. 4,723,048; and *Pollitzer* U.S. 3,780,122.

A pre-reaction start-up procedure comprising passing a hot, inert gas (hydrogen or nitrogen) across the catalyst and reactor bed prior to feedstock introduction is disclosed in *Butler et al*., U.S. Patent No. 4,956,511 and U.S. Patent No. 4,665,258 also to *Butler et al*. Another pre-reaction, start-up procedure aimed at controlling the hygroscopic tendency of mordenite involves subjecting the catalyst to a dry calcination procedure as disclosed in U.S. Patent No. 4,151,120 to *Marcilly*. Conventional TDP processes utilizing Ni-Mordenite catalysts suffer from numerous disadvantages, including their sensitivity and susceptibility to being traumatized by ammonia, moisture, temperature changes, plant power failures, and other changes in operating conditions. Also, conventional Ni-mordenite catalysts suffer from the disadvantage of requiring up to three or more days to get lined out during reactor setup, during which period of startup, there is no significant acceptable production across the catalyst. The present invention provides a TDP process utilizing a Ni-mordenite catalyst that overcomes these deficiencies while allowing the production rates to be increased several fold and the selectivities to be improved at the same time.

### SUMMARY OF THE INVENTION

In accordance with the present invention, there is provided a process for the disproportionation of a substantially pure toluene feedstock over a dealuminated nickel-mordenite catalyst wherein increased production rates of up to two times normal commercial rates can be achieved at lower temperatures, utilizing lower reactor pressures, and benefiting from improved selectivities, without detrimental effect on catalyst activity or catalyst life.

### BRIEF DESCRIPTION OF THE DRAWING

Figure 1 is a graph illustrating toluene conversion in a disproportionation process carried out over a nickel modified mordenite catalyst illustrating temperature and pressure over time.

Figure 2 is a graph of the toluene disproportionation process displaying catalyst aromatics selectivity at different pressures over time.

Figure 3 illustrates the heavies and gas selectivities for the TDP process.

### DESCRIPTION OF THE PREFERRED EMBODIMENTS

As evident in the patents previously discussed, the use of nickel modified catalysts in toluene disproportionation is well known in the art The present invention, however, provides an improved mode of conducting toluene disproportionation over a nickel modified mordenite catalyst wherein the catalyst has been selected to allow significant increases in the production rates and improvements in selectivity, utilizing lower plateau temperature, without loss of catalyst activity.

In accordance with the invention, there is provided a toluene disproportionation process employing a catalyst of the mordenite type modified by the inclusion of a metallic hydrogenation component, more specifically nickel. The mordenite catalyst employed in the present invention preferably exhibits a silica-to-alumina mole ratio of between about 10 and about 50, and more preferably about 20. One particularly advantageous Ni-Mordenite catalyst that was found to be useful in the present invention was that commercial catalyst designated as T-2581, manufactured and sold by United Catalyst, Inc., 1227 South 12th Street, Louisville, Kentucky 40210. This catalyst exhibits the following typical physical and chemical properties: form - extrusion; diameter 1.4 to 1.6mm; compacted bulk density of 0.59g/cm³ ± 0.05 (37 +/- 3 lbs/ft³); crush strength (average of 15 pellets) of about 498g (1.1lbs); Hg Pore Volume greater than 0.3 cm³/g ; BET Surface Area exceeding 200m²/g; and % LOI at 538°C (1000°F) below about 8.

The mordenite disproportionation catalyst employed in the present invention has been modified by the inclusion of nickel. Applicant's experimentation suggests that best results are obtained by utilizing a catalyst made up of no less than 0.3 weight percent nickel. It is known that low nickel content mordenite catalysts provide toluene conversion and selectivity to xylenes and benzene but exhibit poor aging qualities. Experimentation has determined that, while greater amounts of nickel can be used without added benefit, a practical upper limit of nickel content in the catalyst is about 1.5 weight percent.

The present invention resulted from studies conducted over a period of approximately 130 days utilizing a laboratory reactor. In the presence of hydrogen gas, a substantially pure toluene feedstock was disproportionated over a nickel modified mordenite catalyst under the following initial conditions: temperature 360 - 432°C (680° - 810°F); mordenite silica-to-alumina molar ratio catalyst of 20 +/-2 weight percent; hydrogen/toluene mole ratio of about 1 up to about 4 feedstock liquid hourly space velocity (LHSV) between 1.0-4; and at an inlet pressure that varied between 34.5 bars (500 psig) and 41.4 bars (600 psig). The reaction temperature is initially 360°C (680°F) and is thereafter raised to 432°C (810°F).

The present invention further discloses a method of forming benzene and xylene from a toluene feedstock comprising flowing said feedstock across a bed of nickel-mordenite catalyst in the presence of hydrogen at a temperature sufficient to produce a predetermined desirable conversion rate of the feedstock into xylenes at a liquid hourly space velocity of 2hr⁻¹; thereafter increasing the liquid hourly space velocity of the feedstock to 4hr⁻¹ and simultaneously increasing the hydrogen feed in amounts sufficient to maintain the same molar ratio of hydrogen to hydrocarbons as initially present in the toluene/hydrogen feed, and additionally simultaneously increasing the temperature of the reaction sufficiently to maintain saic predetermined desirable conversion rate.
In said process,
- the initial H : HC ratio is 1 : 1 ; it is then raised and maintained at 4 : 1 ;
- the predetermined conversion rate is between 46 and 47 percent ;
- the pressure is 34.5 bars (500 psig) ;
- the liquid hourly space velocity is increased to 6 hr⁻¹ ;
- the temperature is raised from an initial temperature of 360°C (680°F) to a temperature of 432°C (810°F).

The present process was devised in a laboratory reactor utilizing the aforementioned catalyst designated as T-2581. It had previously been thought that the commercial space velocity of the TDP reaction was generally limited to about 2hr⁻¹ at inlet at pressures in the catalyst chamber of 41.4 bars (600 psig). The laboratory reactor was set up to simulate the same reaction obtained in the commercial reactor by inserting in the laboratory reactor the above described commercially available nickel mordenite catalyst. The laboratory reactor test comprised loading catalyst into a 2.53 cm (1 inch) lab reactor having 0.635 cm (1/4 inch) thermowell. Pretreatment was performed on the catalyst at the same pressure as the test run. After the lab reactor was loaded and pressure checked, it was installed in the flow system. Hydrogen flow was started at 0.8 liters per minute and adjusted to the reactor test pressure, either 34.5 bars (500 psig) or 41.4 bars (600 psig).
The temperature in the reactor was ramped upward to 250°C. Thereafter, the toluene feed was added to the reactor and the hydrogen flow was adjusted to obtain a hydrogen to hydrocarbon ratio of 1:1 (molar).

It was found that the production rate of the reactor could be increased several-fold without adversely affecting the catalyst life, the selectivity, or the activity. During the first run through the reactor, base conditions were established that corresponded to conventional commercial conditions. Those conditions were LHSV = 2.0hr⁻¹;
Pressure = 41.4 bars (600 psig) (inlet);
Temperature = adjusted to obtain 47% toluene conversion;
Hydrogen rate relative to hydrocarbon = initial 1:1 (molar ratio); Subsequent 4:1 (molar ratio).
After the initial base conditions above were established in the laboratory reactor, a base case run of the reactor was performed over a time period of 88 days. A second test run was performed with new catalyst under the same base conditions except that the LHSV was doubled to 4hr⁻¹. This test run extended for a period of about 50 days. A third test run was then performed over a fresh charge of catalyst but at a reduced reactor pressure of 34.5 bars (500 psig), at an LHSV of 4hr⁻¹, for a period of 130 days.

The temperature of all catalyst test runs was adjusted in order to provide a 47% conversion of toluene. Hydrogen flow was increased after the nonaromatic selectivity had fallen below the level of about 2.0 to 2.5%. For the two test runs at 34.5 bars (500 psig) this was done at 15 and 20 days. For the 41.4 bars (600 psig) test, the hydrogen rate was adjusted on the ninth day.

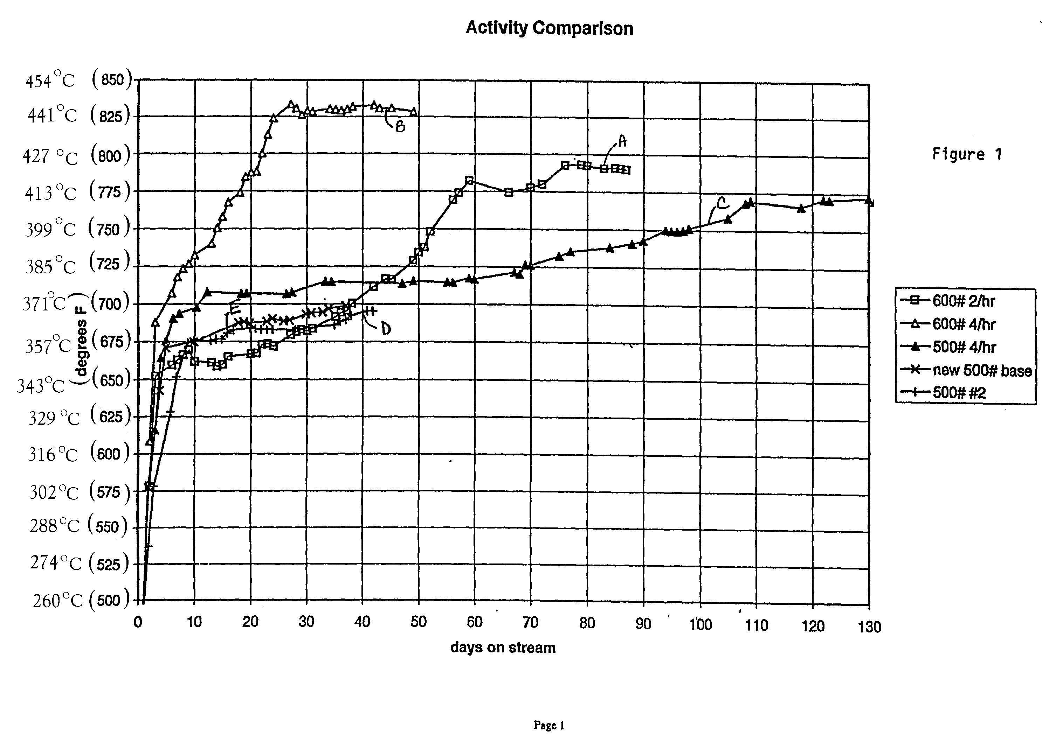

Figure 1 illustrates a plot of temperature versus time to indicate the activity of the catalyst during the test runs. The base case for the nickel mordenite catalyst was done at 41.4 bars (600 psig) and an LHSV of 2/hr. This run is indicated on Figure 1 as line A. In the base case, a plateau temperature of approximately 418°C (785°F) was achieved at about 60 days on stream. Several days later the base case temperature was adjusted to 421°C (790°F) in order to obtain the desired conversion rate of 47%.

A second run at 41.4 bars (600 psig) and an LHSV of 4/hr is indicated in Figure 1 by line B. The plateau temperature in this run was approximately 19.4°C (35°F) higher than the base case, leveling off at approximately 440.6 - 446°C (825-835°F). It is believed that the 19.4 (35°) temperature difference was a consequence of additional heat required by the kinetics of the reaction to keep conversion constant for the high space velocity test. Also, the temperature plateau for the high space velocity test at 41.4 bars (600 psig) was reached in half the time of the lower space velocity test run. It appears then that catalyst aging is a function of the barrels of toluene feed processed and not time. When measured on a barrel of toluene feed per pound of catalyst basis, the temperature plateau is reached at almost identically the same point.

The third test which was performed at a reactor inlet pressure of 34.5 bars (500 psig) and a flow rate of 4 per hour is indicated in Figure 1 as line C. Initially, the temperature required to obtain a 47% toluene conversion level was between the two 41.4 bars (600 psig) tests. Since the runs started at 5.5 - 8.3°C (10-15°F) lower than the other high velocity test (4/hr) there was some activity advantage in operating at the 34.5 bars (500 psig) level. Shortly after startup, it became obvious that the catalyst was more stable under the 34.5 bars (500 psig) conditions. The deactivation rate at 34.5 bars (500 psig) was slower than both of the previous 41.4 bars (600 psig) tests. This even included the 41.4 bars (600 psig) test done at the lower space velocity (2/hr) which is half of the space velocity of test C. The third test at 34.5 bars (500 psig) took approximately 110 days to reach a temperature plateau of 410°C (770°F). This was about twice as long as the 41.4 bars (600 psig) 2/hr base case required to reach a plateau which was 11.1 °C (20°F) higher.

The behavior of these three tests is entirely contrary to what would be expected by the expert in toluene disproportionation, based on a hydrogen partial pressure reaction model. It is believed that the behavior can be explained by considering the catalyst performance with respect to pressure, being constantly mindful of the partial pressure of the products and the reactants. It is further believed that the most likely component of the system to condense is the heavies fraction which is produced as a side product. It is envisioned that the condensation of heavies inside the catalyst pores occurs at a much greater extent at 41.4 bars (600 psig) than it does at 34.5 bars (500 psig). Therefore, it is likely that condensation of heavy hydrocarbon liquid in the catalyst pores is a precursor to coking and subsequent deactivation of the catalyst. A coking mechanism such as this would also have a negative effect on selectivity since a byproduct of coking is extra gas production. The capillary condensation model is useful to explain the current performance of the laboratory reactor experiments.

The advantages of this unexpected result are obvious once the result was observed. One would have expected that the commercial unit operating at a higher space velocity would run fewer calendar days than it had at the standard space velocity. Since operation at 34.5 bars (500 psig) actually results in lower deactivation rates and less coking on the catalyst, this lower operating pressure presents an opportunity to increase the flow velocity over the catalyst without decreasing the overall time that the catalyst is active. Two additional laboratory tests were then conducted to redefine a base case using a reactor pressure of 34.5 bars (500 psig) and an LHSV of 3/hr. These two tests are represented in Figure 1 as lines D and E. This result in Figure 1 illustrates that the deactivation rate of the catalyst during the latest 34.5 bars (500 psig) runs roughly parallels the deactivation rate of the 34.5 bars (500 psig) run at a space velocity of 4/hr. Both of the later 34.5 bars (500 psig) tests cross the temperature plot of the 44,4 bars (600 psig) test. Thus, these two catalyst modes were deactivating slower than the 41.4 bars (600 psig) base case, i.e., 0.33°C (0.6°F) per day versus 1.33°C (2.4°F) per day.

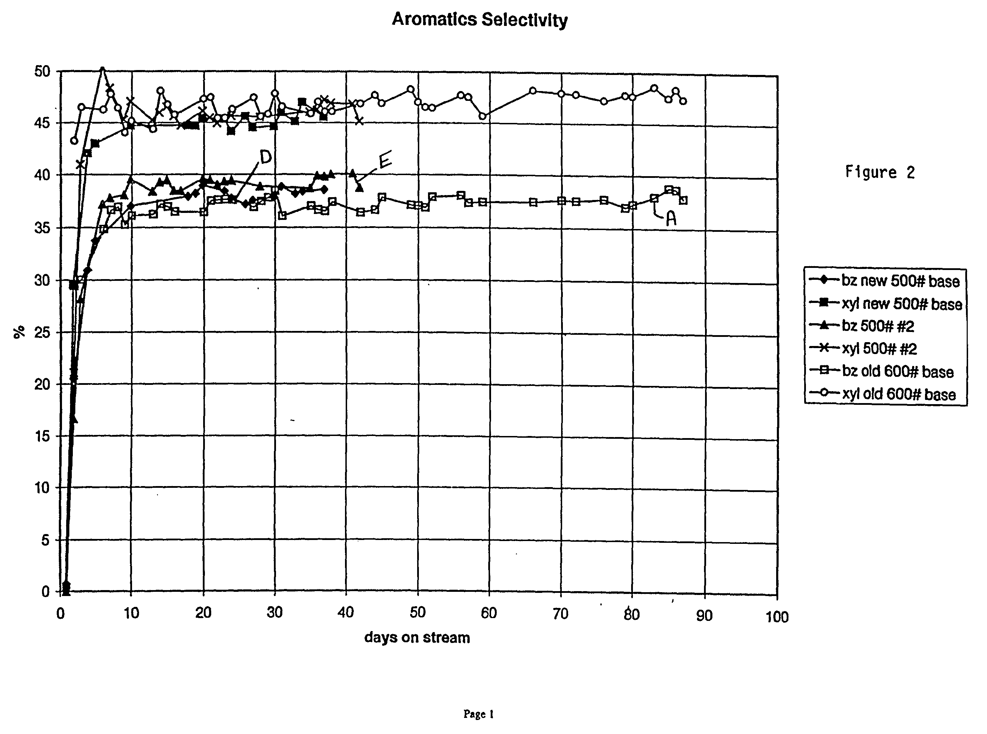

Figure 2 illustrates a representation of aromatic selectivity based on days on stream of the reactor. The test runs indicated on Figure 2 include the two later 34.5 bars (500 psig) runs at an LHSV of 3/hr, as well as the base case run at 41.4 bars (600 psig) and an LHSV of 2/hr. The three test runs represented by the three lines at the top of Figure 2 all indicate xylene selectivity from each of the separate test conditions, i.e., the two 34.5 bars (500 psig) cases and the base case 41.4 bars (600 psig) case. This shows that the xylene selectivity was nearly the same for all cases, and that any differences in xylene selectivity were merely within the limits of the tests themselves.
However, for benzene selectivity, a measurable difference exists between the various tests, there being an improvement in the 34.5 bars (500 psig) benzene selectivity of about 2% over the 41.4 bars (600 psig) benzene selectivity. In Figure 2, line A represents the 41.4 bars (600 psig) test run and the other two lines represent the two 34.5 bars (500 psig) test runs, i.e., lines D and E. The benzene selectivity of the 41.4 bars (600 psig) test run averaged approximately 37% while the benzene selectivity of the two 34.5 bars (500 psig) runs averaged about 39%. This is a considerable improvement considering that the tests were all done on the same catalyst.

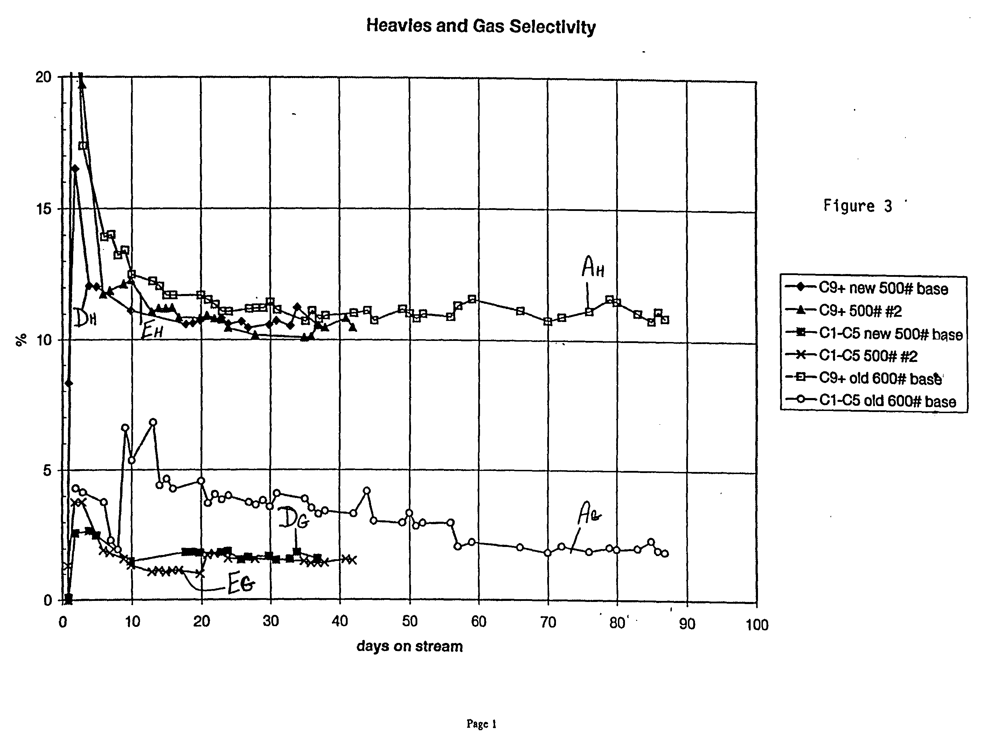

Figure 3 indicates where the additional benzene was obtained in the test runs. The difference in selectivity to gases (C1 - C5) was about 2% where the two 34.5 bars (500 psig) test runs produced approximately 2% less gas than the 41.4 bars (600 psig) test. This shift from gas production to benzene production is a desirable result and improves the production of desirable products through the disproportionation unit. In a normal commercial reactor, a 2% increase in selectivity of benzene and a reduction of gas production of 2% can result in an increase in profits of several million dollars per year. It should also be noted that in Figure 3 a small reduction in selectivity to heavies production (C₉+) amounting to approximately ½ percent was obtained by running at the 34.5 bars (500 psig) pressure. In Figure 3, the upper three lines represent the heavies selectivities and the lower three lines represent the gas selectivities. The various runs are labeled A_{H}, D_{H} and E_{H} for heavies production and A_{G}, D_{G} E_{G} for gas selectivities.

In conclusion, running the toluene disproportionation reactor at 34.5 bars (500 psig) rather than 41.4 bars (600 psig) resulted in a slower aging of the catalyst and a desirable reduction of approximately 11.1°C (20°F) in the plateau temperature. The tests run at 34.5 bars (500 psig) also resulted in selectivity improvement to benzene of approximately 2% which by itself could result in millions of dollars in increased production profits over a one year period. This increase in benzene selectivity was obtained by a corresponding decrease in gas selectivity of approximately 2%, which is a desirable result. Although not shown, a test was performed starting at 41.4 bars (600 psig) and running for several days then reducing the reactor pressure to 34.5 bars (500 psig) to determine the optimum time for adjusting the reactor inlet pressure. This test did not show any significant improvement over a normal run at a constant 41.4 bars (600 psig). Therefore, it is believed that the coking and capillary action of the heavies on deactivating the catalyst occurs almost immediately after the reactor is started and therefore the lower reactor pressures should be utilized from the start-up of the new catalyst. It is believed that the additional production of benzene is a significant economic advantage to justify the present invention. However, even additional justifications can be found in the lower plateau temperatures and the slower deactivation rates of the catalyst which would allow the unit to run longer and more efficient before the catalyst must be changed.

It has also been shown graphically and experimentally that running the reactor at the reduced pressure allows the volumetric throughput (LHSV) to be increased without deteriorating or degrading the activity of the catalyst. This also results in increasing production rates through the disproportionation unit which provides economic incentive for the present invention.

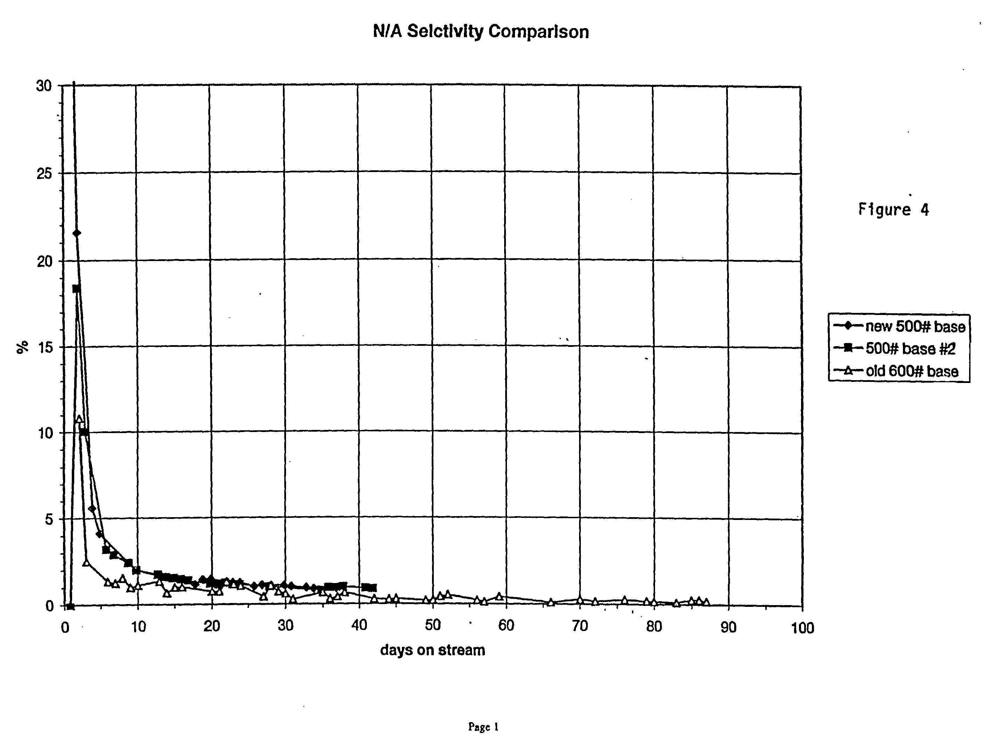

Thus, it has been graphically and experimentally shown by the description above given with respect to Figures 1-4 that, with the catalyst described herein, there is no dramatic negative effect on the catalyst when activity is measured on the volumetric basis, a reduced pressure is used in the reactor, and the throughput rates are increased two-fold over conventional rates. When throughput rates were increased from 2hr⁻¹ to 4hr⁻¹ at the reduced reactor pressure, a better benzene selectivity and improvement in nonaromatic selectivity was achieved, in addition to the doubling of production rates through the TDP unit. It was also found that xylene isomer selectivity did not change significantly with increased throughputs. Also, doubling the space velocity did not increase the deactivation rate at the start of run when measured on a volumetric basis.

## Claims

1. A process for forming benzene and xylene from a toluene feedstock comprising flowing said feedstock in the presence of hydrogen across a bed of nickel-mordenite catalyst in the presence of hydrogen, under intial conditions **characterized by** a temperature of from 360 °C to 427°C ( 680°F to 800°F), a silica to alumina molar ratio of 20 +/- 2, a hydrogen to toluene molar ratio of from 1 to 4, a LHSV of the feedstock of from 1 to 4 hr ⁻¹ and an inlet pressure of from 34,5 to 41,4 bars (500 to 600 psig).

2. The process of claim 1 wherein said inlet pressure is maintained at a pressure of 34.5 bar.

3. The process of claim 2 where said liquid hourly space velocity of the feedstock is maintained at 4 hr ⁻¹ and the hydrogen feed simultaneously adjusted to maintain a toluene to hydrogen molar ratio of 4 .

4. The process any of claim 1 to 3 wherein said catalyst has a dealuminated nickel-mordenite structure having an extruded form with a size between 1.4 mm and 1.6 mm, a compacted bulk density of between 0.54 and 0.64 g/cm³ ( 34 and 40 lbs/ft³), a 15 pellets average crush strength of at least 498 g ( 1.1 lbs), an Hg pore volume greater than 0.3 cm³/g, a BET surface area exceeding 200 m²/g, a percent LOI at 538 °C ( 1000 °F) below about 8 and a nickel content of no less than 0.3 weight %.

## Patentansprüche

1. Verfahren zum Bilden von Benzol und Xylol aus einem Toluoleinsatzmaterial, umfassend Fließen lassen des Einsatzmaterials in der Anwesenheit von Wasserstoff über ein Bett von Nickel-Mordenit Katalysator in der Anwesenheit von Wasserstoff unter Anfangsbedingungen, **gekennzeichnet durch** eine Temperatur von 360°C bis 427°C (680°F bis 800°F), einem Silica zu Aluminiumoxid Molverhältnis von 20 +/-2, einem Wasserstoff zu Toluol Molverhältnis von 1 bis 4, einer LHSV des Einsatzmaterials von 1 bis 4 h⁻¹ und einem Einlaßdruck von 34,5 bis 41,4 Bar (500 bis 600 psig).

2. Verfahren nach Anspruch 1, **dadurch gekennzeichnet, daß** der Einlaßdruck bei einem Druck von 34,5 Bar gehalten wird.

3. Verfahren nach Anspruch 2, **dadurch gekennzeichnet, daß** die stündliche Flüssigkeitsraumgeschwindigkeit des Einsatzmaterials bei 4h⁻¹ gehalten wird, und die Wasserstoffbeschickung gleichzeitig unter Beibehalten eines Toluol zu Wasserstoff Molverhältnisses von 4 eingestellt wird.

4. Verfahren nach Anspruch 1 bis 3, **dadurch gekennzeichnet, daß** der Katalysator eine entaluminierte Nickel-Mordenit Struktur mit einer extrudierten Form mit einer Größe zwischen 1,4 mm und 1,6 mm, einer Stampfdichte zwischen 0,54 und 0,64 g/cm³ (34 und 40 Pfund/Fuß³), einer 15 Pellet Durchschnittbruchstärke von mindestens 498 g (1,1 Pfund), einem Hg Porenvolumen größer als 0,3 cm³/g, einem BET Oberflächenbereich, der 200 m²/g übersteigt, einem Prozent LOI bei 538°C (1000°F) unterhalb etwa 8 und einem Nickelgehalt von nicht weniger als 0,3 Gew.% hat.

## Revendications

1. Procédé pour former du benzène et du xylène à partir d'une charge de départ de toluène comprenant les étapes de faire passer cette charge de départ en présence d'hydrogène au travers d'un lit de catalyseur de nickel-mordénite en présence d'hydrogène, sous des conditions initiales, **caractérisé par** une température de 360°C à 427°C (680°F à 800°F), un rapport molaire silice à alumine de 20 +/- 2, un rapport molaire d'hydrogène à toluène de 1 à 4, une LHSV de la charge de départ de 1 à 4 hr⁻¹ et une pression d'entrée de 34,5 à 41,4 bars (500 à 600 psig).

2. Procédé selon la revendication 1 où cette pression d'entrée est maintenue à une pression de 34,5 bars.

3. Procédé selon la revendication 2 où cette vitesse spatiale horaire à l'état liquide de la charge de départ est maintenue à 4 hr⁻¹ et l'alimentation en hydrogène est ajustée simultanément pour maintenir un rapport molaire toluène à hydrogène de 4.

4. Procédé selon l'une quelconque des revendications 1 à 3 où ce catalyseur a une structure désaluminée de nickel-mordénite ayant une forme extrudée avec une dimension entre 1,4 mm et 1,6 mm, une masse volumique apparente à l'état compact entre 0,54 et 0,64 g/cm³ (34 et 40 lbs/ft³), une cohésion moyenne de 15 granules d'au moins 498 g (1,1 lbs), un volume de pores Hg supérieur à 0,3 cm³/g, une aire de surface BET dépassant 200 m²/g, un pourcentage LOI à 538°C (1000°F) inférieur à environ 8 et une teneur en nickel ne dépassant pas 0,3% en poids.
